# EUROPÄISCHE PATENTSCHRIFT

(11) **EP 3 747 558 B1**
(45) Veröffentlichungstag und Bekanntmachung des Hinweises auf die Patenterteilung: **06.09.2023**
(21) Anmeldenummer: 20178492.3
(22) Anmeldetag: 05.06.2020
(51) Int. Cl.: B08B 3/12, A61B 90/70, G02B 23/24, G01N 21/01, B08B 7/02, H04N 23/51, H04N 23/52, G01N 21/15, G02B 27/00

(54) **VORRICHTUNG, MASCHINE ODER MASCHINENKOMPONENTE MIT DER VORRICHTUNG, VERFAHREN ZUR ÜBERWACHUNG MITTELS DER VORRICHTUNG UND VERFAHREN ZUR REINIGUNG DER VORRICHTUNG**
DEVICE, MACHINE OR MACHINE COMPONENT WITH THE DEVICE, METHOD FOR MONITORING BY MEANS OF THE DEVICE AND METHOD FOR CLEANING THE DEVICE
DISPOSITIF, MACHINE OU COMPOSANT DE MACHINE DOTÉ DU DISPOSITIF, PROCÉDÉ DE SURVEILLANCE AU MOYEN DU DISPOSITIF ET PROCÉDÉ DE NETTOYAGE DU DISPOSITIF

(30) Priorität: 07.06.2019 DE 102019115564
(43) Veröffentlichungstag der Anmeldung: 09.12.2020
(73) Patentinhaber: MML Solutions GmbH, 46487 Wesel (DE)
(72) Erfinder: Mtauweg, Samer, 27568 Bremerhaven (DE); Reglin, Alexej, 26655 Westerstede (DE); Meimann, Valentin, 48429 Rheine (DE); Lin, Ying, 46487 Wesel (DE)
(74) Vertreter: advotec.

(56) Entgegenhaltungen:
- EP-A1- 1 765 003
- DE-A1-102014 200 219
- FR-A1- 3 052 416
- US-A1- 2017 361 360
- US-A1- 2018 245 490

## Beschreibung

Die Erfindung betrifft eine Vorrichtung, insbesondere zur optischen Überwachung von Maschinen oder Maschinenkomponenten. Ferner betrifft die Erfindung eine Maschine oder Maschinenkomponente mit der Vorrichtung. Außerdem betrifft die Erfindung ein Verfahren zur Reinigung der Vorrichtung.

Aus dem Stand der Technik sind Vorrichtungen zur optischen Überwachung von Maschinen oder Maschinenkomponenten seit langem bekannt. Zur Überwachung werden dabei insbesondere bildgebende Verfahren eingesetzt, die sich mithilfe von Kameras oder Endoskopen auf einfache Weise eine Bildgebung ermöglichen.

Aus der DE 10 2014 200219 A1 ist eine Vorrichtung zur Reinigung eines optischen Elements eines Kamerasystems bekannt. Die FR 3 052 416 A1 beschreibt eine Vorrichtung zum Schutz eines optischen Sensors und ein optisches Erkennungssystem für ein Kraftfahrzeug. In der US 2017/361360 A1 ist ein Ultraschall-Linsenreinigungssystem beschrieben. Weiterer Stand der Technik ist der EP 1 765 003 A1 und der US 2018 / 245 490 A1 zu entnehmen.

Nachteilig an den bekannten Vorrichtungen ist, dass Linsen der Vorrichtungen mit Dampf, Nebeldampf, Ölen, Öldämpfen, Ölblasen oder sonstigem Schmutz bedeckt bzw. kontaminiert werden, sobald sie zum Einsatz kommen. Konsequenterweise ist eine regelmäßig manuelle Reinigung erforderlich. Die Reinigung gestaltet sich jedoch zeitaufwändig und damit kostenintensiv. Ohne eine entsprechende Reinigung kann eine Sichtbehinderung vorliegen, die eine optische Überwachung langfristig unmöglich macht.

Der Erfindung liegt daher die Aufgabe zugrunde, die aus dem Stand der Technik bekannten Nachteile zu überwinden.

Diese Aufgabe ist durch die Vorrichtung mit den Merkmalen des Anspruchs 1, durch die Maschine oder Maschinenkomponente mit den Merkmalen des Anspruchs 10 und durch das Verfahren mit den Merkmalen des Anspruchs 11 gelöst. Vorteilhafte Weiterbildungen der Erfindung sind in den Unteransprüchen angegeben.

Zur Vermeidung von Wiederholungen sollen vorrichtungsgemäß offenbarte Merkmale auch als verfahrensgemäß offenbart gelten.

Ebenso sollen verfahrensgemäß offenbarte Merkmale auch als vorrichtungsgemäß offenbart gelten.

Erfindungsgemäß wird eine Vorrichtung vorgeschlagen, die insbesondere zur optischen Überwachung von Maschinen oder Maschinenkomponenten eingesetzt wird. Die Vorrichtung umfasst ein Gehäuse mit einem im Gehäuse angeordneten und mindestens eine Linse umfassenden Bildaufnahmegerät, wobei zur Vermeidung von Verschmutzungen der Linse die Vorrichtung mindestens ein Ultraschallgerät zum Emittieren von Ultraschallwellen in eine Umgebung der Vorrichtung umfasst und/oder die Vorrichtung mindestens ein weiteres Ultraschallgerät zum Emittieren von Ultraschallwellen auf die Linse umfasst.

Durch das Vorsehen des mindestens einen Ultraschallgeräts ist es möglich, Schmutz aus der Umgebung, insbesondere dem Blickfeld des Bildaufnahmegeräts, zu beseitigen bzw. entfernen. Weiter ist es möglich durch das Vorsehen des mindestens einen weiteren Ultraschallgeräts die Linse des Geräts von möglichen Verschmutzungen zu reinigen. Damit kann mittels der vorgeschlagenen Ultraschallapplikation Schmutz aus der Umgebung von der Vorrichtung und damit der Linse ferngehalten oder beseitigt werden und/oder bei Kontamination der Linse diese von Verschmutzungen gereinigt werden.

Unter Verschmutzungen können Kontaminationen verstanden werden. Weiterhin kann unter Verschmutzungen Schmutz, Dampf, Nebeldampf, Aerosole, Gase, Öle, Öldampf, Ölblasen oder sonstiger Schmutz verstanden werden.

Unter der Umgebung kann ein Raum verstanden werden, der die Vorrichtung umgibt. Mit anderen Worten kann die Umgebung ein Raum oder Bereich außerhalb der Vorrichtung sein.

Unter einem Ultraschallgerät kann eine Ultraschallvorrichtung oder ein Ultraschallmittel verstanden werden, das eingerichtet ist Ultraschallwellen zu emittieren. Sobald Ultraschallwellen auf Schmutz oder Verschmutzungen treffen, werden diese aufgelöst, entfernt bzw. beseitigt. Unter einem Emittieren von Ultraschallwellen kann ein Abgeben, Abstrahlen, Aussenden, Applizieren, etc. von Ultraschallwellen verstanden werden.

Unter einem Bildaufnahmegerät kann ein Bildaufnahmemittel verstanden werden. Das Bildaufnahmegerät kann als Kamera oder als Endoskop ausgebildet sein. Das Bildaufnahmegerät kann innerhalb eines Lagers, vorzugsweise einer Hauptlagerung eines Getriebes und/oder eines Generators, einer zu untersuchenden Maschine eingebracht werden.

Das Bildaufnahmegerät kann zur Aufnahme von sequenten Bildern (2D und/oder 3D) und/oder Videos ausgebildet sein. Darüber hinaus kann die Bildaufnahme bei ausgewählten Betriebszuständen, wie zum Beispiel beim Trudelbetrieb einer Anlage, manuell oder aus der Ferne erfolgen. Bei diesen Betriebszuständen ist mit weniger Aerosol und Verschmutzungen in der Umgebung und dem Blickfeld des Aufnahmegeräts vor und/oder während der Bildaufnahme zu rechnen.

Nach einer entsprechenden Ausrichtung des Bildaufnahmegeräts und/oder der Linse können Videoaufnahmen und/oder in der Abhängigkeit der anliegenden Drehzahl des zu untersuchenden Maschinenelements sequente Bilder aufgenommen werden.

Die Steuerung der Bild- oder Videoaufnahmen kann entweder aus direkter Nähe unmittelbar am Bildaufnahmegerät selbst und/oder aus der Ferne, zum Beispiel über eine Anlagensteuerung und SCADA-System, erfolgen.

Eine Verwendung von 3D-Kameras und/oder von Schattenlinienprojektionen mit einer Kamera sind denkbar zur Integration in einem Aufnahmekopf zwecks 3D-Messung der Zieloberfläche, um Schäden zu quantifizieren und gegebenenfalls durch Tragbildertests und/oder Lastverteilung und/oder durch Berechnung der Lastaufteilung aus den Messdaten zu erhalten.

Vorzugsweise weist die Vorrichtung ein Mittel auf, um das Bildaufnahmegerät im Gehäuse zu steuern bzw. zu verlagern oder zu verdrehen. Zur Steuerung können beispielsweise mechanische Bauteile, wie Seile oder Verbindungsarme, eingesetzt werden. Über entsprechend vorgegebene Biegewinkel lassen sich die mechanischen Bauteile dann auf einfache Weise verstellen.

Eine Steuerung zum Emittieren der Ultraschallwellen kann in einem elektronischen Bauteil, insbesondere Chip, entweder in dem Bildaufnahmegerät selbst oder innerhalb des Gehäuses integriert sein. Eine entsprechende Ultraschallsteuerung kann über eine kabellose Schnittstelle (beispielsweise Bluetooth oder W-LAN) Daten zu einer zentralen Steuerung übermitteln und empfangen, beispielsweise zwecks Vorgaben von einstellbaren Steuerparametern.

Das mindestens eine Ultraschallgerät kann entweder manuell oder automatisch in Abhängigkeit von Sensordaten, insbesondere sichtbezogenen Sensordaten, gesteuert bzw. angesteuert werden. Die Sensordaten können Informationen umfassen, welche mittels physikalischen Größen die Sichtverhältnisse in der Umgebung des Bildaufnahmegeräts, insbesondere im Blickfeld, beschreiben. So kann beispielsweise die Menge des Nebeldampfes sensorbasiert erfasst werden und das mindestens eine Ultraschallgerät entsprechend gesteuert werden. Steuerbefehle können die Aktivierung und Deaktivierung der Ultraschallerzeugung, deren Signalstärke oder die zeitliche Koordination einzelner Ultraschallgeräte bezüglich Aktivierung, Deaktivierung und Signalstärke sein.

Bevorzugt ist das Bildaufnahmegerät kabellos oder alternativ kabelgebunden ausgeführt. Entsprechendes gilt auch für das mindestens eine Ultraschallgerät. Hierdurch ist es möglich, eine Datenübertragung zur Vorrichtung und von der Vorrichtung weg entweder kabellos (W-LAN, Bluetooth etc.) und/oder kabelgebunden auszuführen.

Weiter bevorzugt ist das mindestens eine Ultraschallgerät mit dem Bildaufnahmegerät in einem gemeinsamen Gerätegehäuse angeordnet. Das Gehäuse kann einen runden, einen ovalen, einen n-eckigen Querschnitt, wobei n größer/gleich drei ist, oder einen aus unterschiedlichen Grundformen zusammengesetzten Querschnitt aufweisen. Das Gehäuse kann zumindest teilweise zylindrisch, insbesondere hohlzylindrisch und/oder rohrartig ausgebildet sein. Dadurch ist es möglich, die verschiedenen Komponenten der Vorrichtung im Gehäuse zu integrieren oder am Gehäuse anzuordnen.

Besonders bevorzugt ist das Gehäuse als ein flexibles Rohr ausgebildet. Hierdurch lässt sich das Rohr bei Bedarf biegen oder verformen.

Die Ultraschallwellen können einerseits auf die Linse des Bildaufnahmegeräts und/oder andererseits wahlweise bei Bedarf in die Umgebung des Bildaufnahmegeräts abgestrahlt werden. Hierfür kann ein einziges oder auch mehrere Ultraschallgerät(e) verwendet werden. Eine Entscheidungsgröße hierfür kann der Abstrahlwinkel der Ultraschallwellen sein. Jedem Ultraschallgerät kann eine separate Abstrahlrichtung über eine räumliche Orientierung der Linse relativ zur Umgebung zugewiesen werden.

Vorzugsweise umfasst die Vorrichtung mindestens oder genau zwei Ultraschallgeräte, wobei zum Emittieren von Ultraschallwellen in die Umgebung die Vorrichtung ein erstes Ultraschallgerät umfasst, und/oder zum Emittieren von weiteren Ultraschallwellen auf die Linse die Vorrichtung ein zweites Ultraschallgerät umfasst.

Durch das Vorsehen der mindestens zwei Ultraschallgeräte ist es auf einfache Weise möglich, Schmutz aus der Umgebung zu beseitigen und die Linse, im Fall von Verschmutzungen, effektiv zu reinigen.

Bevorzugt ist das mindestens eine Ultraschallgerät parallel zur Blickrichtung des Bildaufnahmegeräts am Gehäuse angeordnet, und/oder das weitere Ultraschallgerät ist im Gehäuse angeordnet. Hierdurch ist ein verbesserter Reinigungseffekt erzielbar.

Weiter bevorzugt ist das mindestens eine Ultraschallgerät stirnseitig oder mantelseitig am Gehäuse angeordnet und/oder das weitere Ultraschallgerät ist in einem Bereich zwischen einer Stirnseite des Gehäuses und dem Bildaufnahmegerät angeordnet. Hierdurch ist ein verbesserter Reinigungseffekt erzielbar.

Bevorzugt umfasst die Vorrichtung vier Ultraschallgeräte, wobei zum Emittieren von Ultraschallwellen in die Umgebung die Vorrichtung ein erstes und ein zweites Ultraschallgerät umfasst und/oder wobei zum Emittieren von weiteren Ultraschallwellen auf die Linse die Vorrichtung ein drittes und viertes Ultraschallgerät umfasst. Durch das Vorsehen von vier Ultraschallgeräten ist es auf einfache Weise möglich, Schmutz aus der Umgebung noch besser zu beseitigen und die Linse, im Fall von Verschmutzungen, noch effektiver zu reinigen.

Vorzugsweise sind dabei das erste und zweite Ultraschallgerät parallel zur Blickrichtung des Bildaufnahmegeräts an einem Ende des Gehäuses angeordnet. Bevorzugt sind das dritte und vierte Ultraschallgerät einander gegenüberliegend im Gehäuse angeordnet.

Die Vorrichtung umfasst einen Blindstopfen, in dem das Gehäuse angeordnet oder integriert ist. Mit anderen Worten kann der Blindstopfen gehäusemantelseitig am Gehäuse anliegen.

Durch das Vorsehen des Blindstopfens ist es auf einfache Weise möglich, die Vorrichtung bei der optischen Überwachung von Maschinen oder Maschinenkomponenten entsprechend an Gehäuseteilen bzw. Komponententeilen der Maschinen zu fixieren bzw. zu positionieren.

Der Blindstopfen kann beispielsweise in einem Lagergehäuse oder in einem Endoskopiedeckel oder dem Gehäuse eines Getriebes, insbesondere während der Aufnahme der Rotation einer Maschine, eingesetzt werden. Hierdurch sind Leckagen vermeidbar.

Erfindungsgemäß umfasst das Gehäuse einen Blindstopfen und einen am Gehäuse angeordneten bzw. fixierten Flansch (auch Fixierungsflansch genannt), wobei der Flansch eingerichtet ist, den Blindstopfen in seiner Längserstreckung parallel zur Längserstreckung des Gehäuses zu positionieren. Durch das Vorsehen des Flansches wird eine Möglichkeit geschaffen, den Blindstopfen relativ zum Flansch zu verlagern. Dabei kann der Flansch als ein Ring, ein O-Ring, ein Ring mit Innengewinde, eine Hohlschraube oder eine Scheibe ausgebildet sein.

Vorzugsweise weist die Vorrichtung wenigstens eine Dichtung auf, die zwischen dem Flansch und dem Blindstopfen angeordnet ist.

Weiter vorzugsweise weist die Vorrichtung wenigstens ein Befestigungsmittel auf, das den Flansch und den Blindstopfen verbindet. Als Befestigungsmittel können Stifte, Klammern und/oder Schrauben, etc. verwendet werden.

Erfindungsgemäß weist der Flansch ein Gewinde auf, wobei der Blindstopfen ein das Gewinde kämmendes Gegengewinde aufweist. Durch das Vorsehen des Gewindes und des Gegengewindes lässt sich der Blindstopfen relativ zum Flansch verlagern und positionieren.

Alternativ erfindungsgemäß weist das Gehäuse ein Gewinde auf, wobei der Flansch ein das Gewinde kämmendes Gegengewinde aufweist. Auch hier ist eine relative Verlagerung erzielbar.

Besonders bevorzugt ist jeweils eine Schraubenmutter beidseitig des Flansches angeordnet, wobei die Schraubenmuttern jeweils ein mit dem Gewinde des Gehäuses kämmendes Gegengewinde aufweisen. Hierdurch wird eine weitere Möglichkeit bereitgestellt, den Blindstopfen relativ zum Flansch zu verlagern und zu positionieren.

Vorzugsweise kann bei einer Vorrichtung, die einen Blindstopfen umfasst, wie oben beschrieben, auf sämtliche Ultraschallgeräte der Vorrichtung verzichtet werden.

Das Gehäuse kann als ein Endoskopiekanal ausgebildet sein. Vorzugsweise ist der Endoskopiekanal ein Rohr, insbesondere ein flexibles Rohr. Durch das Vorsehen des Gehäuses als Endoskopiekanal ist es möglich, die Vorrichtung zur optischen Überwachung von Maschinen oder Maschinenkomponenten weit genug in die entsprechenden Maschinen oder Maschinenkomponenten einzuführen.

Wie oben bereits erwähnt, kann das Bildaufnahmegerät als Kamera oder als Endoskop ausgebildet sein.

Für einen flexiblen Einsatz der Vorrichtung kann eine Änderung des Anschlusses des Gerätegehäuses von dem Bildaufnahmegerät zum Einsatzort gegebenenfalls zu einem Endoskopiedeckel einer Endoskopielücke einfach realisierbar sein. Vorteilhaft ist, dass anstatt eines festen Einbaus eine optische Überwachung variabel an unterschiedlichen Messstellen erfolgen kann. Weiter vorteilhaft kann das Bildaufnahmegerät aus dem Gehäuse entnehmbar sein und in ein entsprechend anders dimensioniertes Gerät in einfacher und reversibler Weise eingesetzt werden. So kann ein einziges Bildaufnahmegerät für unterschiedliche Messstellen verwendet werden.

Eine Zentralsteuerung für mehrere automatisierbare Endoskope kann die Ultraschallsteuerung alternativ oder zusätzlich übernehmen. Anhand übermittelter oder berechneter Drehzahlen bzw. des lokalen Drehwinkels lässt sich eine Vorgabe, beispielsweise für die Frequenz der Bildaufnahme, an den einzelnen automatisierten Endoskopen definieren. Dies kann in Abhängigkeit der Drehzahl und der Maschinenkinematik ermittelt werden.

Weiter erfindungsgemäß wird eine Maschine oder Maschinenkomponente umfassend mindestens eine oben beschriebene Vorrichtung vorgeschlagen.

Damit kann die Vorrichtung in einer bspw. durch Öl, Dämpfe oder Flüssigkeiten verschmutzten Umgebung zur Überwachung von Maschinen und Maschinenkomponenten sowohl im Stillstand als auch im Betrieb, wie bspw. bei rotatorischen oder translatorischen Bewegungen, der Maschinen und Maschinenkomponenten erfolgen.

Vorzugsweise ist die Vorrichtung in einem Lager, Getriebe, Rohr oder Verrohrungssystem der Maschine oder der Maschinenkomponente anordenbar oder angeordnet. Die Einsatzmöglichkeiten sind nicht abschließend.

Insbesondere kann die Vorrichtung in bzw. innerhalb von Rohren oder Verrohrungssystemen der Maschinen oder Maschinenkomponenten eingesetzt werden. Ein Beispiel hierfür kann eine Ölverrohrung eines Getriebes sein, welche einerseits unabhängig von der Applikation der Vorrichtung bei jedem Getriebe benötigt wird und bei welcher andererseits die Positionierung von Spritzdüsen bzw. Schmiermittel-Abflussbohrungen der Verrohrungssysteme optimal zur Bildaufnahme an aktiven Flächen der Komponenten liegt.

Vorzugsweise ist die Vorrichtung in einem Lager, Getriebe, Rohr oder Verrohrungssystem der Maschine oder der Maschinenkomponente anordenbar oder angeordnet. Die Einsatzmöglichkeiten sind nicht abschließend.

Weiter erfindungsgemäß wird ein Verfahren zur Reinigung oder zum Sauberhalten einer Vorrichtung vorgeschlagen, die insbesondere zur optischen Überwachung von Maschinen und/oder Maschinenkomponenten eingesetzt wird. Mittels des Verfahrens sind die nachfolgenden Schritte realisierbar:
- Emittieren von Ultraschallwellen in eine Umgebung der Vorrichtung mittels mindestens eines Ultraschallgeräts der Vorrichtung und/oder
- Emittieren von weiteren Ultraschallwellen mittels mindestens eines weiteren Ultraschallgeräts auf mindestens eine Linse eines Bildaufnahmegeräts der Vorrichtung.

Durch das Vorsehen des Verfahrens ist es möglich, Schmutz aus der Umgebung, insbesondere dem Blickfeld des Bildaufnahmegeräts, zu beseitigen bzw. entfernen. Weiter ist es möglich durch das Vorsehen des mindestens einen weiteren Ultraschallgeräts die Linse des Geräts von möglichen Verschmutzungen zu reinigen.

Damit kann mittels der vorgeschlagenen Ultraschallapplikation Schmutz aus der Umgebung von der Vorrichtung und damit der Linse ferngehalten oder beseitigt werden und/oder bei Kontamination der Linse diese von Verschmutzungen gereinigt werden. Weitere Merkmale der Erfindung ergeben sich aus der nachfolgenden Beschreibung von bevorzugten Ausführungsbeispielen der Erfindung in Verbindung mit den Zeichnungen und den Unteransprüchen. Hierbei können die einzelnen Merkmale für sich allein oder in Kombination miteinander verwirklicht sein.

In den Zeichnungen zeigen:
- Figur 1: eine bekannte Vorrichtung aus dem Stand der Technik;
- Figur 2: eine weitere bekannte Vorrichtung aus dem Stand der Technik;
- Figur 3: eine erste Ausführungsform einer erfindungsgemäßen Vorrichtung;
- Figur 4: eine zweite Ausführungsform einer erfindungsgemäßen Vorrichtung;
- Figur 5: eine dritte Ausführungsform einer erfindungsgemäßen Vorrichtung;
- Figur 6: eine vierte Ausführungsform einer erfindungsgemäßen Vorrichtung;
- Figur 7: eine fünfte Ausführungsform einer erfindungsgemäßen Vorrichtung;
- Figur 8: eine sechste Ausführungsform einer erfindungsgemäßen Vorrichtung;
- Figur 9: eine siebte Ausführungsform einer erfindungsgemäßen Vorrichtung;
- Figur 10: einen Schnitt durch ein beliebiges Lager mit einem eingesetzten Blindstopfen;
- Figur 11: das Lager aus Figur 10 mit einer erfindungsgemäßen Vorrichtung;
- Figur 12: einen Schnitt durch ein zweistufiges Getriebe;
- Figur 13: das Getriebe aus Figur 12 mit einer erfindungsgemäßen Vorrichtung;
- Figur 14: eine perspektivische Ansicht von Komponenten der Vorrichtung;
- Figur 15: einen Längsschnitt durch die Komponenten aus Figur 14;
- Figur 16: einen Längsschnitt gemäß Figur 15 mit einem Endoskopiekanal;
- Figur 17: eine erste Ausführungsform eines Endoskopiekanals;
- Figur 18: eine zweite Ausführungsform des Endoskopiekanals; und
- Figur 19: eine dritte Ausführungsform des Endoskopiekanals.

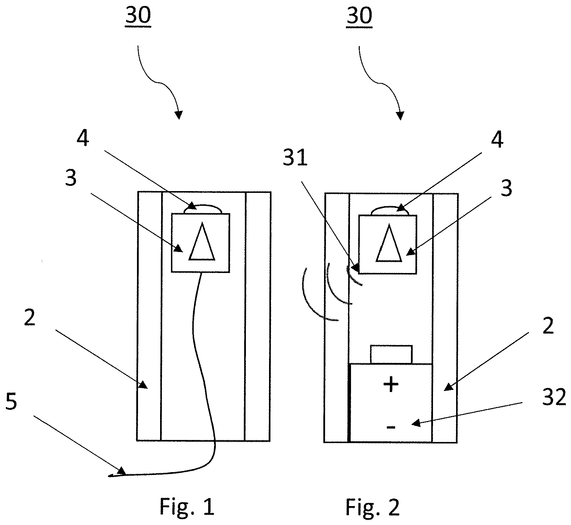

Figur 1 zeigt eine aus dem Stand der Technik bekannte Vorrichtung 30. Die Vorrichtung 30 umfasst ein Gehäuse 2. Im Gehäuse 2 ist ein Bildaufnahmegerät 3 angeordnet. Das Bildaufnahmegerät 3 weist eine Linse 4 auf. Die Vorrichtung 30 ist kabelgebunden.

Hierzu ist eine Leitung 5 am Bildaufnahmegerät 3 zur kabelgebundenen Datenübertragung angeschlossen.

Figur 2 zeigt eine weitere aus dem Stand der Technik bekannte Vorrichtung 30. Im Unterschied zu der Vorrichtung 30 aus Figur 1 ist das Bildaufnahmegerät 3 gemäß Figur 2 nicht kabelgebunden, sondern wird mittels einer Stromquelle in Form eines Akkumulators 32 mit Strom versorgt. Darüber hinaus ist das Bildaufnahmegerät 3 eingerichtet, eine kabellose Datenübertragung mittels einer Datenschnittstelle 31 auszuführen.

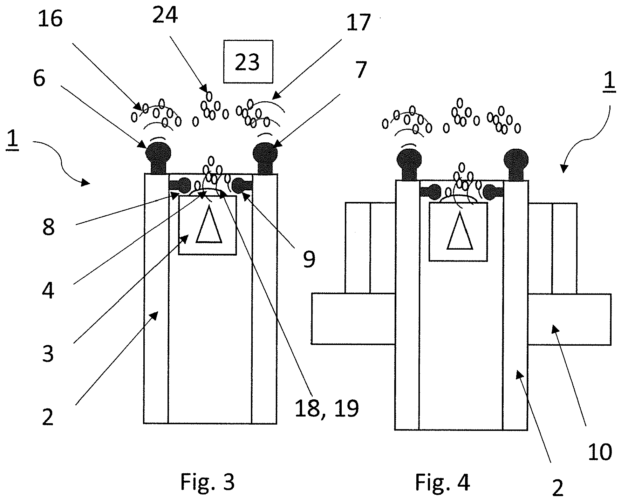

Figur 3 zeigt eine Vorrichtung 1. Die Vorrichtung 1 befindet sich in einer mit Schmutz oder Verschmutzungen 24 versehenen Umgebung 23. Die Vorrichtung 1 ist insbesondere zur optischen Überwachung von Maschinen oder Maschinenkomponenten eingerichtet. Die Vorrichtung 1 umfasst ein Gehäuse 2 mit einem im Gehäuse 2 angeordneten und eine Linse 4 umfassenden Bildaufnahmegerät 3. Ferner umfasst die Vorrichtung vier Ultraschallgeräte 6, 7, 8, 9. Zum Emittieren von ersten Ultraschallwellen 16, 17 in die Umgebung 23 sind ein erstes und ein zweites Ultraschallgerät 6, 7 parallel zur Blickrichtung des Bildaufnahmegeräts 3 an einem Ende des Gehäuses 2 angeordnet. Das erste und das zweite Ultraschallgehäuse 6, 7 sind dabei stirnseitig am Gehäuse 2 angebracht. Das Gehäuse 2 ist hohlzylindrisch ausgebildet.

Zum Emittieren von weiteren Ultraschallwellen 18, 19 auf die Linse 4 sind ein drittes und viertes Ultraschallgerät 8, 9 einander gegenüberliegend im Gehäuse angeordnet. Dabei sind das dritte und das vierte Ultraschallgerät 8, 9 in einem Bereich zwischen der Stirnseite des Gehäuses 2 und dem Bildaufnahmegerät 3 angebracht.

Das Bildaufnahmegerät 3 kann kabellos oder kabelgebunden sein. Entsprechendes gilt auch für die vier Ultraschallgeräte 6, 7, 8, 9.

Durch das Vorsehen der Ultraschallgeräte 6, 7 ist es auf einfache Weise möglich, Schmutz aus der Umgebung 23 besser zu beseitigen bzw. entfernen, um Verunreinigungen der Linse 4 zu vermeiden. Durch das Vorsehen der Ultraschallgeräte 8, 9 ist es auf einfache Weise möglich, Verschmutzungen 24 auf der Linse 4 oder im Bereich der Linse 4, effektiver zu entfernen.

Damit kann mittels der vorgeschlagenen Ultraschallapplikation Schmutz 24 aus der Umgebung 23 von der Vorrichtung 1 und damit der Linse 4 ferngehalten oder beseitigt werden und bei Kontamination der Linse 4, diese von Verschmutzungen 24 gereinigt werden.

Figur 4 zeigt eine weitere Ausführungsform einer Vorrichtung 1. Zusätzlich zur Vorrichtung 1 aus Figur 3 umfasst die Vorrichtung 1 gemäß Figur 4 einen Blindstopfen 10. Der Blindstopfen 10 umgibt das Gehäuse 2 der Vorrichtung 1. Durch das Vorsehen des Blindstopfens 10 ist es möglich die Vorrichtung 1 bei der optischen Überwachung von Maschinen oder Maschinenkomponenten entsprechend an Gehäuseteilen bzw. Komponententeilen der Maschinen zu fixieren bzw. zu positionieren.

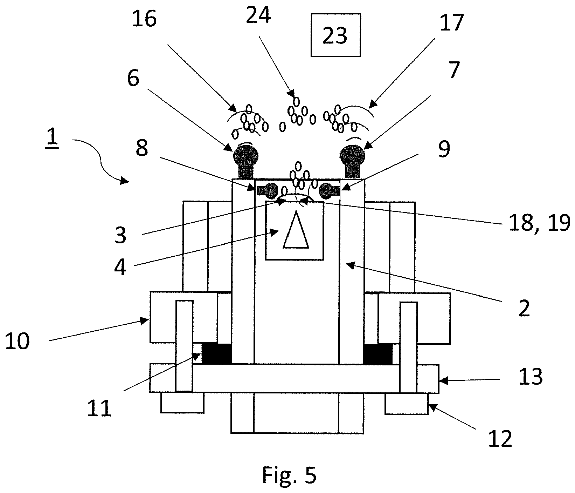

Figur 5 zeigt eine weitere Ausführungsform einer Vorrichtung 1. Im Unterschied zur Figur 3 weist die Vorrichtung 1 gemäß Figur 5 eine lösbare Verbindung aus Schrauben 12 (oder anderen Mitteln, wie Klemmen) zwischen dem Gehäuse 2 und dem Blindstopfen 10 auf. Zwischen dem Gehäuse 2 und dem Blindstopfen 10 ist ferner eine Dichtung 11 (beispielsweise in Form einer Ringdichtung) angeordnet. Bei der gezeigten Verbindung durchgreifen mehrere Schrauben 12 einen Flansch 13 des Gehäuses 2 und greifen in einen Flansch des Blindstopfens 10 ein. Hierdurch ist das Gehäuse 2 des Aufnahmegeräts gegen axiales Rutschen sicherbar.

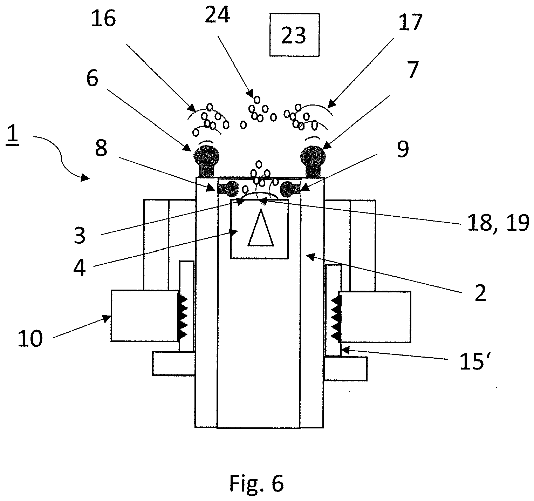

Figur 6 zeigt eine weitere Ausführungsform einer Vorrichtung 1. Im Unterschied zu der Vorrichtung gemäß Figur 5 ist eine alternative Verbindung, mit einer als Flansch ausgebildeten Hohlschraube 15' gezeigt.

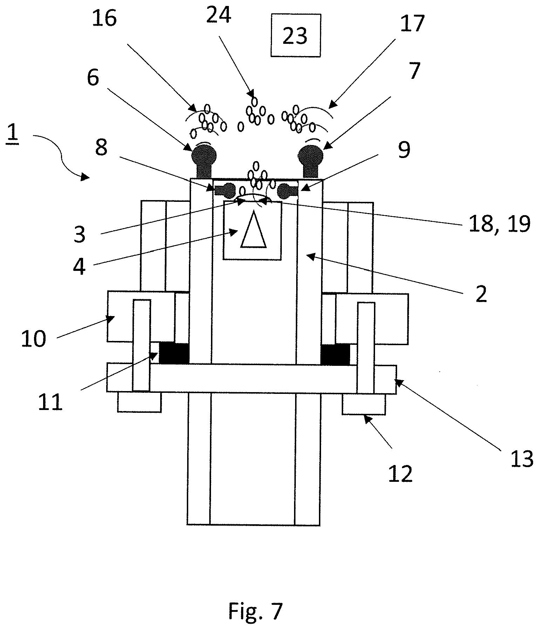

Figur 7 zeigt eine weitere Ausführungsform einer Vorrichtung 1. Im Unterscheid zur Figur 5 umfasst die Vorrichtung 1 gemäß Figur 7 ein verlängertes Gehäuse 2 zur Aufnahme der Befestigungsmittel 12.

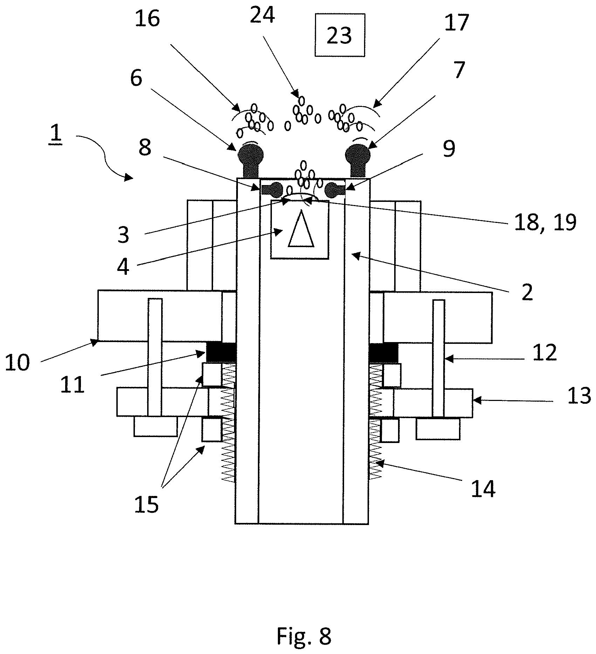

Figur 8 zeigt eine weitere Ausführungsform einer Vorrichtung 1. Im Unterscheid zur Figur 5 umfasst die Vorrichtung 1 gemäß Figur 8 einen als Fixierungsflansch 13 ausgebildeten Ring, ein am Gehäuse 2 angeordnetes Gewinde 14 zur axialen Einstellbarkeit des Gehäuses 2 und zur axialen Sicherung gegen Verrutschen oder Herausrutschen, sowie zwei in das Gewinde 14 eingreifende Schraubenmuttern 15 zur axialen Sicherung gegen Verrutschen.

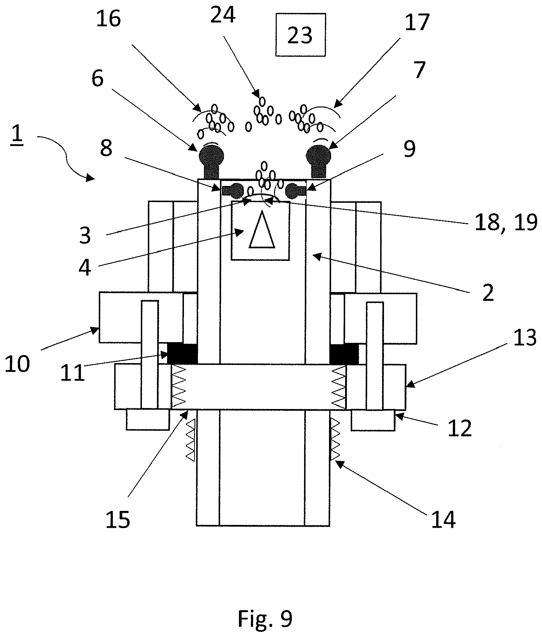

Figur 9 zeigt eine weitere Ausführungsform einer Vorrichtung 1. Im Unterschied zur Figur 5 umfasst die Vorrichtung 1 gemäß Figur 9 ein Gewinde 14, das am Gehäuse 2 zur axialen Verschiebbarkeit und axialen Sicherung gegen ein Herausrutschen über ein Sicherungselement, in Form einer Schraubenmutter 15 angeordnet ist. Ferner umfasst der Fixierungsflansch 13 ein Innengewinde, welches zum Eingreifen in das Gewinde des Gehäuses 2 ausgebildet ist.

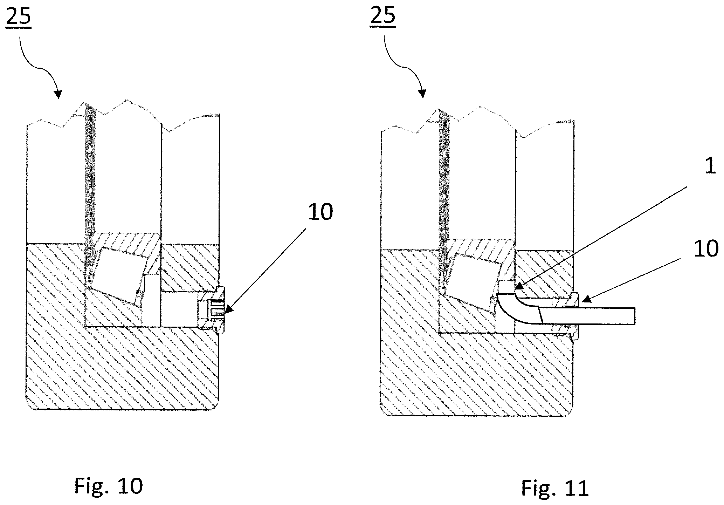

Figur 10 zeigt einen Schnitt durch ein beliebiges Lager 25 mit einem Lagergehäuse. In einem Bereich des Lagergehäuses ist ein Blindstopfen 10 in eine Öffnung eingesetzt.

Figur 11 zeigt das Lager 25 aus Figur 10 und eine erfindungsgemäße Vorrichtung 1.

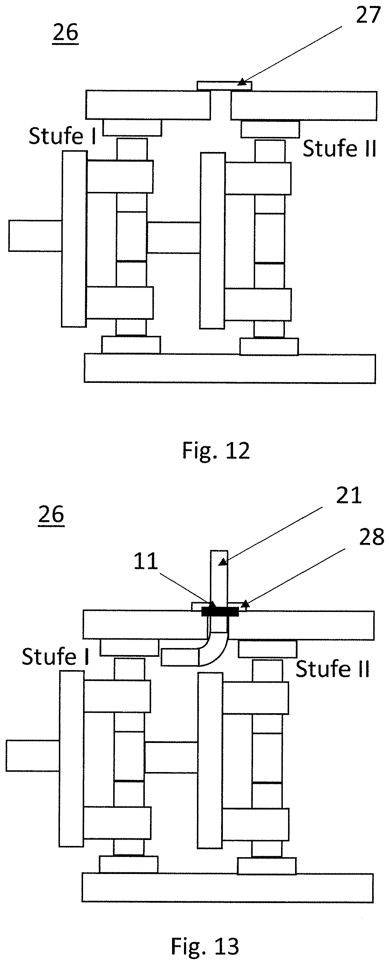

Figur 12 zeigt einen Schnitt durch ein zweistufiges Getriebe 26 mit einem Getriebegehäuse. In einem Bereich des Lagergehäuses ist ein Deckel 27 zum Verschließen einer Gehäuseöffnung angeordnet.

Figur 13 zeigt das Getriebe 26 aus Figur 12 ohne den Deckel 27, aber mit einer Dichtung 11, einem Ersatzdeckel 28 mit Öffnung und einen als Gehäuse ausgebildeten flexiblen Endoskopiekanal 21, der durch den Ersatzdeckel 28 und die Dichtung geführt ist. Der Ersatzdeckel kann ein Blindstopfen sein.

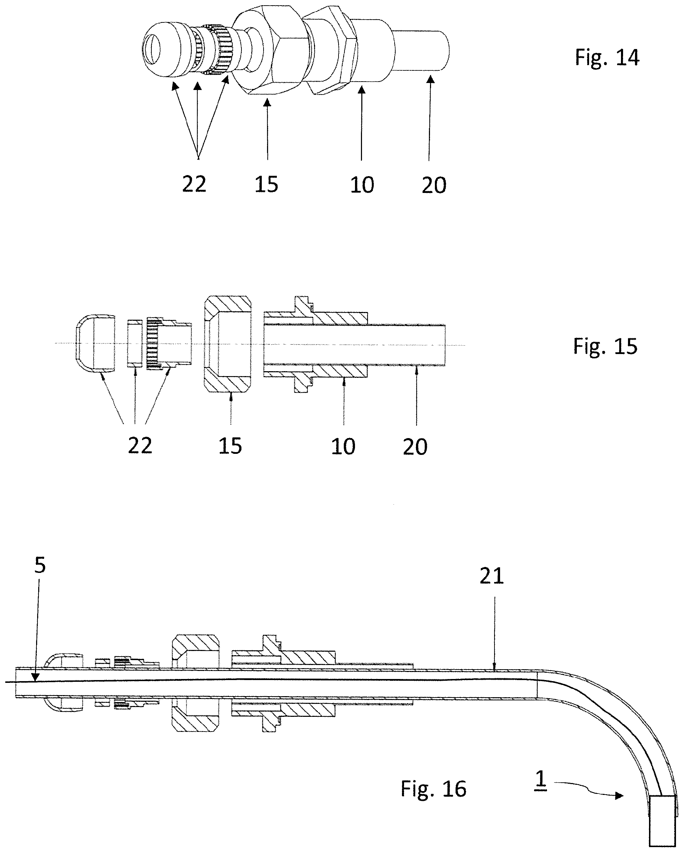

Die Figuren 14 und 15 zeigen Komponenten der erfindungsgemäßen Vorrichtung. Dabei weist ein Blindstopfen 10 eine Führung 20 auf, wobei die Führung 20 vom Blindstopfen 10 zumindest teilweise umgriffen wird. Zwischen den Komponenten einer Schraubenverbindung 22 und dem Blindstopfen 10 ist eine Schraubenmutter 15 zur Verbindung der Schraubenverbindung 22 und dem Blindstopfen 10 angeordnet.

Figur 16 zeigt die Komponenten gemäß den Figuren 14 und 15, wobei das Gehäuse 2 der Vorrichtung als ein Endoskopiekanal 21 durch die Komponenten geführt ist.

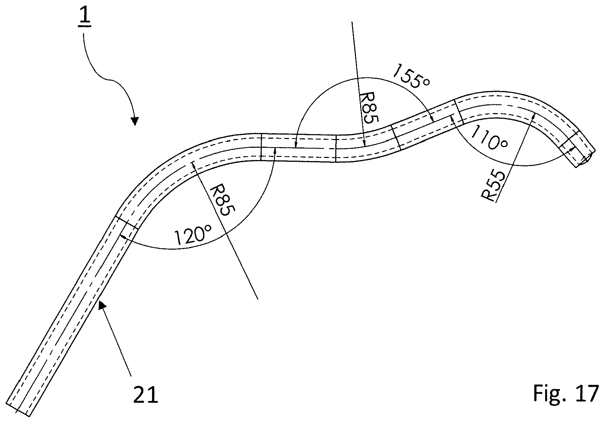

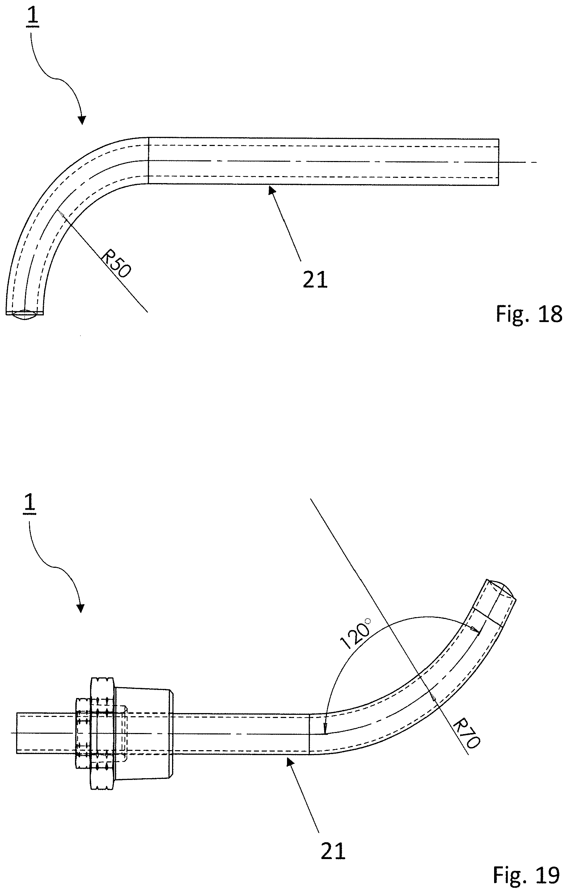

In den Figuren 17, 18 und 19 sind unterschiedliche Ausführungsformen bzw. Ausgestaltungen des Endoskopiekanals 21 gezeigt. Die gezeigten Radien und Winkel sind nur exemplarisch.

### Bezugszeichenliste

- 1: Vorrichtung
- 2: Gehäuse
- 3: Bildaufnahmegerät
- 4: Linse
- 5: Kabel/Leitung
- 6: Ultraschallgerät
- 7: Ultraschallgerät
- 8: Ultraschallgerät
- 9: Ultraschallgerät
- 10: Blindstopfen
- 11: Dichtung
- 12: Befestigungsmittel
- 13: Flansch (Fixierungsflansch)
- 14: Gewinde
- 15: Schraubenmutter
- 15': Hohlschraube
- 16: Ultraschallwellen
- 17: Ultraschallwellen
- 18: Ultraschallwellen
- 19: Ultraschallwellen
- 20: Führung
- 21: Endoskopiekanal
- 22: Schraubverbindung
- 23: Umgebung
- 24: Schmutz oder Verschmutzungen
- 25: Lager
- 26: Getriebe
- 27: Endoskopiedeckel
- 28: Ersatzdeckel
- 30: Vorrichtung aus dem Stand der Technik
- 31: Datenschnittstelle
- 32: Akkumulator (Batterie)

## Patentansprüche

1. Vorrichtung (1), insbesondere zur optischen Überwachung von Maschinen oder Maschinenkomponenten, umfassend ein Gehäuse (2) mit einem im Gehäuse (2) angeordneten und mindestens eine Linse (4) umfassenden Bildaufnahmegerät (3), wobei zur Vermeidung von Verschmutzungen der Linse (4) die Vorrichtung (1) mindestens ein Ultraschallgerät (6, 7) zum Emittieren von Ultraschallwellen (16, 17) in eine Umgebung (23) der Vorrichtung (1) umfasst, und/oder die Vorrichtung (1) mindestens ein weiteres Ultraschallgerät (8, 9) zum Emittieren von Ultraschallwellen (18, 19) auf die Linse (4) umfasst, wobei die Vorrichtung (1) einen Blindstopfen (10), in dem das Gehäuse (2) angeordnet oder integriert ist, und einen am Gehäuse (2) fixierten Flansch (13) umfasst, wobei der Flansch (13) eingerichtet ist, den Blindstopfen (10) in seiner Längserstreckung parallel zur Längserstreckung des Gehäuses (2) zu positionieren, wobei das Gehäuse (2) ein Gewinde aufweist und der Flansch (13) ein das Gewinde kämmendes Gegengewinde aufweist und/oder wobei jeweils eine Schraubenmutter (15) beidseitig des Flansches (13) angeordnet ist, und die Schraubenmuttern (15) jeweils ein mit dem Gewinde des Gehäuses (2) kämmendes Gegengewinde aufweisen.

2. Vorrichtung (1) nach Anspruch 1, wobei das mindestens eine Ultraschallgerät (6, 7) parallel zur Blickrichtung des Bildaufnahmegeräts (3) am Gehäuse (2) angeordnet ist, und/oder das weitere Ultraschallgerät (8, 9) im Gehäuse (2) angeordnet ist.

3. Vorrichtung (1) nach einem der vorherstehenden Ansprüche, wobei das mindestens eine Ultraschallgerät (6, 7) stirnseitig oder mantelseitig am Gehäuse (2) angeordnet ist und/oder das weitere Ultraschallgerät (8, 9) in einem Bereich zwischen einer Stirnseite des Gehäuses (2) und dem Bildaufnahmegerät (3) angeordnet ist.

4. Vorrichtung (1) nach einem der vorherstehenden Ansprüche, wobei das Gehäuse (2) zylindrisch und/oder rohrförmig ist.

5. Vorrichtung (1) nach einem der vorherstehenden Ansprüche, umfassend vier Ultraschallgeräte (6, 7, 8, 9), wobei zum Emittieren von Ultraschallwellen (16, 17) in die Umgebung (23) die Vorrichtung (1) ein erstes und ein zweites Ultraschallgerät (6, 7) umfasst, und wobei zum Emittieren von weiteren Ultraschallwellen (18, 19) auf die Linse (4) die Vorrichtung (1) ein drittes und viertes Ultraschallgerät (8, 9) umfasst, wobei insbesondere das dritte und vierte Ultraschallgerät (8, 9) einander gegenüberliegend im Gehäuse (2) angeordnet sind.

6. Vorrichtung (1) nach einem der Ansprüche 1 bis 5, wobei die Vorrichtung (1) wenigstens eine Dichtung (11) aufweist, die zwischen dem Flansch (13) und dem Blindstopfen (10) angeordnet ist.

7. Vorrichtung (1) nach einem der Ansprüche 1 bis 6, wobei die Vorrichtung (1) wenigstens ein Befestigungsmittel (12) aufweist, das den Flansch (13) und den Blindstopfen (10) verbindet und/oder wobei der Flansch (13) ein Gewinde aufweist und der Blindstopfen (10) ein das Gewinde kämmendes Gegengewinde aufweist.

8. Vorrichtung (1) nach einem der vorherstehenden Ansprüche, wobei das Gehäuse (2) als Endoskopiekanal (21) ausgebildet ist.

9. Vorrichtung (1) nach einem der vorherstehenden Ansprüche, wobei das Bildaufnahmegerät (3) als Kamera oder als Endoskop ausgebildet ist.

10. Maschine oder Maschinenkomponente umfassend mindestens eine Vorrichtung (1) nach einem der vorherstehenden Ansprüche, wobei die Vorrichtung (1) insbesondere in einem Lager (25), Getriebe (26), Rohr oder Verrohrungssystem der Maschine oder der Maschinenkomponente anordenbar oder angeordnet ist.

11. Verfahren zur Reinigung einer Vorrichtung (1) nach einem der Ansprüche 1 bis 9 umfassend die folgenden Schritte:
- Emittieren von Ultraschallwellen (16, 17) in eine Umgebung (23) der Vorrichtung (1) mittels mindestens eines Ultraschallgeräts (6, 7) der Vorrichtung (1) und/oder
- Emittieren von weiteren Ultraschallwellen (18, 19) mittels mindestens eines weiteren Ultraschallgeräts (8, 9) der Vorrichtung (1) auf die mindestens eine Linse (4) des Bildaufnahmegeräts (3) der Vorrichtung (1).

## Claims

1. A device (1), in particular for optically monitoring machines or machine components, the device (1) comprising a housing (2) having an image acquisition device (3), the image acquisition device (3) being disposed in the housing (2) and comprising at least one lens (4), the device (1) comprising at least one ultrasonic device (6, 7) for emitting ultrasonic waves (16, 17) into an environment (23) of the device (1) for avoiding an accumulation of dirt on the lens (4), and/or the device (1) comprising at least one another ultrasonic device (8, 9) for emitting ultrasonic waves (18, 19) onto the lens (4), the device (1) comprising a blind plug (10), in which the housing (2) is disposed or integrated, and a flange (13) attached to the housing (2), the flange (13) being configured to position the blind plug (10) parallel to the longitudinal dimension of the housing (2) in the longitudinal dimension of the blind plug (10), the housing (2) having a thread, and the flange (13) having a mating thread meshing with said thread, and/or a screw nut (15) being disposed on either side of the flange (13), the screw nuts (15) each having a mating thread meshing with the thread of the housing (2).

2. The device (1) according to claim 1, wherein the at least one ultrasonic device (6, 7) is disposed on the housing (2) parallel to the line of sight of the image acquisition device (3), and/or the other ultrasonic device (8, 9) is disposed in the housing (2).

3. The device (1) according to any one of the preceding claims, wherein the at least one ultrasonic device (6, 7) is disposed on the front or on the wall of the housing (2), and/or the other ultrasonic device (8, 9) is disposed in an area between a front of the housing (2) and the image acquisition device (3).

4. The device (1) according to any one of the preceding claims, wherein the housing (2) is cylindrical and/or tubular.

5. The device (1) according to any one of the preceding claims, the device (1) comprising four ultrasonic devices (6, 7, 8, 9), the device (1) comprising a first and a second ultrasonic device (6, 7) for emitting ultrasonic waves (16, 17) into the environment (23), and the device (1) comprising a third and a fourth ultrasonic device (8, 9) for emitting additional ultrasonic waves (18, 19) onto the lens (4), the third and the fourth ultrasonic device (8, 9) in particular being disposed opposite each other in the housing (2).

6. The device (1) according to any one of claims 1 to 5, wherein the device (1) has at least one seal (11) disposed between the flange (13) and the blind plug (10).

7. The device (1) according to any one of claims 1 to 6, wherein the device (1) has at least one fastening means (12) connecting the flange (13) and the blind plug (10), and/or wherein the flange (13) has a thread, and the blind plug (10) has a mating thread meshing with said thread.

8. The device (1) according to any one of the preceding claims, wherein the housing (2) is an endoscopy channel (21).

9. The device (1) according to any one of the preceding claims, wherein the image acquisition device (3) is a camera or an endoscope.

10. A machine or a machine component comprising at least one device (1) according to any one of the preceding claims, the device (1) being disposed or configured to be disposed in particular in a bearing (25), a gear mechanism (26), a tube or a tubing system of the machine or the machine component.

11. A method for cleaning a device (1) according to any one of claims 1 to 9, the method comprising the following steps:
- emitting ultrasonic waves (16, 17) into an environment (23) of the device (1) by means of at least one ultrasonic device (6, 7) of the device (1); and/or
- emitting additional ultrasonic waves (18, 19) onto the at least one lens (4) of the image acquisition device (3) of the device (1) by means of at least one other ultrasonic device (8, 9) of the device (1).

## Revendications

1. Dispositif (1), destiné notamment à surveiller optiquement des machines ou des composants de machine, le dispositif (1) comprenant un boîtier (2) ayant un appareil de prise d'image (3), l'appareil de prise d'image (3) étant disposé dans le boîtier (2) et comprenant au moins une lentille (4), le dispositif (1) comprenant au moins un appareil à ultrasons (6, 7) pour émettre des ondes ultrasoniques (16, 17) dans un environnement (23) du dispositif (1) afin d'éviter des salissures de la lentille (4), et/ou le dispositif (1) comprenant au moins un autre appareil à ultrasons (8, 9) pour émettre des ondes ultrasoniques (18, 19) sur la lentille (4), le dispositif (1) comprenant un bouchon obturateur (10), dans lequel le boîtier (2) est disposé ou intégré, et une bride (13) fixée au boîtier (2), la bride (13) étant configurée pour positionner le bouchon obturateur (10) parallèlement à la dimension longitudinale du boîtier (2) dans la dimension longitudinale du bouchon obturateur (10), le boîtier (2) ayant un filetage et la bride (13) ayant un contre-filetage engrenant avec ledit filetage et/ou un écrou (15) étant disposé des deux côtes de la bride (13) et les écrous (15) ayant chacun un contre-filetage engrenant avec le filetage du boîtier (2).

2. Dispositif (1) selon la revendication 1, dans lequel l'au moins un appareil à ultrasons (6, 7) est disposé sur le boîtier (2) parallèlement à la direction de vision de l'appareil de prise d'image (3) et/ou l'autre appareil à ultrasons (8, 9) est disposé dans le boîtier (2).

3. Dispositif (1) selon l'une quelconque des revendications précédentes, dans lequel l'au moins un appareil à ultrasons (6, 7) est disposé sur un front ou sur une paroi du boîtier (2) et/ou l'autre appareil à ultrasons (8, 9) est disposé dans une région entre le front du boîtier (2) et l'appareil de prise d'image (3).

4. Dispositif (1) selon l'une quelconque des revendications précédentes, dans lequel le boîtier (2) est cylindrique et/ou tubulaire.

5. Dispositif (1) selon l'une quelconque des revendications précédentes, le dispositif (1) comprenant quatre appareils à ultrasons (6, 7, 8, 9), le dispositif (1) comprenant un premier et un deuxième appareil à ultrasons (6, 7) pour émettre des ondes ultrasoniques (16, 17) dans l'environnement (23), et le dispositif (1) comprenant un troisième et un quatrième appareil à ultrasons (8, 9) pour émettre des ondes ultrasoniques (18, 19) additionnelles sur la lentille (4), le troisième et le quatrième appareil à ultrasons (8, 9) étant disposés notamment en regard l'un de l'autre dans le boîtier (2).

6. Dispositif (1) selon l'une quelconque des revendications 1 à 5, dans lequel le dispositif (1) a au moins un joint d'étanchéité (11) disposé entre la bride (13) et le bouchon obturateur (10).

7. Dispositif (1) selon l'une quelconque des revendications 1 à 6, dans lequel le dispositif (1) a au moins un moyen de fixation (12) reliant la bride (13) et le bouchon obturateur (10) et/ou dans lequel la bride (13) a un filetage et le bouchon obturateur (10) a un contre-filetage engrenant avec ledit filetage.

8. Dispositif (1) selon l'une quelconque des revendications précédentes, dans lequel le boîtier (2) est un canal d'endoscopie (21).

9. Dispositif (1) selon l'une quelconque des revendications précédentes, dans lequel l'appareil de prise d'image (3) est une caméra ou un endoscope.

10. Machine ou composant de machine comprenant au moins un dispositif (1) selon l'une quelconque des revendications précédentes, le dispositif (1) étant disposé ou configuré pour être disposé notamment dans un palier (25), un engrenage (26), un tube ou un système de tuyauterie de la machine ou du composant de machine.

11. Procédé de nettoyage d'un dispositif (1) selon l'une quelconque des revendications 1 à 9, le procédé comprenant les étapes consistant à:
- émettre des ondes ultrasoniques (16, 17) dans un environnement (23) du dispositif (1) au moyen d'au moins un appareil à ultrasons (6, 7) du dispositif (1); et/ou
- émettre des ondes ultrasoniques (18, 19) additionnelles sur l'au moins une lentille (4) de l'appareil de prise d'image (3) du dispositif (1) au moyen d'au moins un autre appareil à ultrasons (8, 9) du dispositif (1).
